# EUROPEAN PATENT SPECIFICATION

(11) **EP 1 855 699 B1**
(45) Date of publication and mention of the grant of the patent: **15.08.2012**
(21) Application number: 05789958.5
(22) Date of filing: 11.03.2005
(51) Int. Cl.: A61K 33/00, A61K 35/02, A61K 47/02

(54) **LIGHT PHARMACEUTICAL WATER AND THERAPEUTIC COMPOSITIONS AND METHODS THEREOF**
LEICHTES PHARMAZEUTISCHES WASSER UND THERAPEUTISCHE ZUSAMMENSETZUNGEN UND VERFAHREN
EAU PHARMACEUTIQUE LEGERE, COMPOSITIONS THERAPEUTIQUES ET PROCEDES CORRESPONDANTS

(43) Date of publication of application: 21.11.2007
(73) Proprietor: Soloviev, Sergey Pavlovich, Moscow 115432 (RU); Timantti AB, 104 30 Stockholm (SE)
(72) Inventor: POMYTKIN, Igor Anatolievich, Moscow, 123557 (RU); SOLOVIEV, Sergey Pavlovich, Moscow, 115432 (RU)
(74) Representative: Hayes, Adrian Chetwynd
(86) International application number: PCT/RU2005/000105
(87) International publication number: WO 2006/098650

(56) References cited:
- WO-A-03/049748
- US-A- 3 451 911
- US-A- 5 855 921
- SOMLYAI G ET AL: "THE BIOLOGICAL EFFECTS OF DEUTERIUM-DEPLETED WATER, A POSSIBLE NEW TOOL IN CANCER THERAPY" DEUTSCHE ZEITSCHRIFT FUER ONKOLOGIE, HEIDELBERG, DE, vol. 30, no. 4, 1998, pages 91-94, XP009006973 ISSN: 0931-0037
- BILD W ET AL: "RESEARCH CONCERNING THE RADIOPROTECTIVE AND IMMUNOSTIMULATING EFFECTS OF DEUTERIUM-DEPLETED WATER" REVUE ROUMAINE DE PHYSIOLOGIE, EDITIONS DE L'ACADEMIE DE LA REPUBLIQUE SOCIALISTE DE LA ROUMA, RO, vol. 36, no. 3/4, July 1999 (1999-07), pages 205-218, XP001145877 ISSN: 0035-399X

## Description

### Technical Field

The present invention relates to human or animal healthcare More specifically, the present invention relates to therapeutic compositions of pharmaceutical water enriched by light water isotopologue ¹H₂¹⁶O.

### Background of the Invention

The pharmaceutical industry places a high priority on the quality of water used in production of finished product, intermediate reagent preparation and analytical processes. Pharmaceutical water quality is regulated by a national pharmacopeia's standards that typically determine the requirements of toxicity, endotoxins, oxidizables, cytotoxicity, and nonvolatile residues. The United States Pharmacopeia (edition USP 23) classifies pharmaceutical water as purified water (PW) and water for injection (WFI). Purified water is used in the process of production of unit dosage form of drugs. Water for injection is water used for preparation of parenterals. Pharmaceutical water is prepared by specific industrial treatment procedures depending of the type of water.

Natural water is a composition of nine water isotopologues (¹H₂¹⁶O, ¹H₂¹⁷O, ¹H₂¹⁸O, ¹H²H¹⁶O, ¹H²H¹⁷O, ¹H²H¹⁸O, ²H₂¹⁶O, ²H₂¹⁷O, and ²H₂¹⁸O) formed by stable isotopes of hydrogen (¹H and ²H) and oxygen (¹⁶O, ¹⁷O, ¹⁸O), wherein the level of light water isotopologue ¹H₂¹⁶O is about 99.7317% (Vienna Standard Mean Ocean Water, VSMOW), and wherein total level of all eight heavy isotopologues comprising at least one heavy isotopes ²H, ¹⁷O, and ¹⁸O is about 0.2683% (e.g. 0.199983% ¹H₂¹⁸O, 0.0372% ¹H₂¹⁷O, 0.031069% ¹H²H ¹⁶O, 0.0000623% ¹H²H ¹⁸O, and 0.0000116% ¹H²H ¹⁷O). Rothman et al., J. Quant. Spectrosc. Radiat. Transfer, 1998, 60, 665. Rothman et al., J. Quant. Spectrosc. Radiat. Transfer, 2003, 82, p.9. The abundance of water isotopologues in natural water slightly varies on Earth district and climatic conditions and is expressed typically as the deviation, δ, relative to the international VSMOW standard. The natural water enriched maximally by major light water isotopologue ¹H₂¹⁶O was founded in Antarctica (Standard Light Antarctic Precipitation, SLAP), wherein said δ-values of residual heavy isotopes are δ²H -415.5‰, δ¹⁷O -28.1‰, and δ¹⁸O -53.9‰ that corresponds to the 99.757 % level of light water isotopologue ¹H₂¹⁶O. R. van Trigt, Laser Spectrometry for Stable Isotope Analysis of Water Biomedical and Paleoclimatological Applications, 2002, Groningen: University Library Groningen, p. 50. Thus, water with the abundance of light water isotopologue ¹H₂¹⁶O more than 99.757% is not found in nature.

Complete depletion of natural water of deuterium-comprising isotopologues (¹H²H¹⁶O, ¹H²H¹⁷O, ¹H²H¹⁸O, ²H₂¹⁶O, ²H₂¹⁷O, and ²H₂¹⁸O) provides water enriched by light water isotopologue ¹H₂¹⁶O to the level never more than 99.76%, since total level of these deuterium-comprising isotopologues in water is below 0.031%. Thus, water with content of light water isotopologue ¹H₂¹⁶O more than 99.76% can be prepared in industrial scale by methods providing depletion of natural water of heavy isotopologues ¹H₂¹⁷O, ¹H₂¹⁸O, ¹H²H¹⁶O, ¹H²H¹⁷O, ¹H²H¹⁸O, ²H₂¹⁶O, ²H₂¹⁷O, and ²H₂¹⁸O.

Pharmaceutical water is prepared from natural water by well-known from the art procedures that do not change isotopic content of the starting water. Accordingly,_pharmaceutical water is the heterogeneous isotopologue composition comprising from about 99.731 to 99.757 % of light water isotopologue ¹H₂¹⁶O and from about 0.243 to 0.269% of residual amounts of heavy isotopologues ¹H₂¹⁷O, ¹H₂¹⁸O, ¹H²H¹⁶O, ¹H²H¹⁷O, ¹H²H¹⁸O, ²H₂¹⁶O, ²H₂¹⁷O, and ²H₂¹⁸O. In order to produce pharmaceutical water with level of light water isotopologue ¹H₂¹⁶O high than 99.760%, it is necessary to deplete natural water of heavy isotopologues ¹H₂¹⁷O, ¹H₂¹⁸O, ¹H²H¹⁶O, ¹H²H¹⁷O, ¹H²H¹⁸O, ²H₂¹⁶O, ²H₂¹⁷O, and ²H₂ ¹⁸O.

Thus, pharmaceutical water with content of light water isotopologue ¹H₂¹⁶O more than 99.757 % is unknown from the art and can be prepared in industrial scale by methods providing a step of depletion of natural water of heavy isotopologues ¹H₂¹⁷O, ¹H₂¹⁸O, ¹H²H¹⁶O, ¹H²H¹⁷O, ¹H²H¹⁸O, ²H₂¹⁶O, ²H₂¹⁷O, and ²H₂¹⁸O. US 58 55 921 discloses pharmaceutical products for aming tumorous discases comprising denterium depleted water.

It is well-documented that light and heavy isotopologues of water have distinct properties and affect distinctly properties of substances in solutions, for example, the rate of biochemical reactions; the enthalpy of association of several binding systems, including protein-carbohydrate, small molecule-small molecule, protein-peptide, and protein-nucleic acid; the thermodynamics (free-energy, enthalpy, entropy and heat-capacity changes) of a ligand binding; the enthalpy of protein unfolding; the thermodynamic stability and formation of functional structures of nucleic acids. Chervenak et al. JACS, 1994, 116 (23): 10533-10539. Makhatadze et al., Nature Struct. Biol., 1995, 2 (10): 852-855. Connelly et al., PNAS, 1994, 91: 1964-1968. Cupane et al., Nucleic Acids Res. 1980, 8 (18): 4283-4303. So, it is well-documented that heavy and light isotopologues of water affect distinctly on basic properties of substances that typically are used as drugs (proteins, nucleic acids, and small molecules). Accordingly, the isotopologue heterogeneity of pharmaceutical water is undesirable and can affect a drug-dose response. Thus, there is the need in monoisotopologous pharmaceutical water in order to produce drug formulations with more advantageous properties.

It is an object of the present invention to provide therapeutic compositions comprising an effective amount of a biologically active agent and a pharmaceutical water, the water content of the composition being from 99.760 to about 99.999% of light isotopologue ¹H₂¹⁶O and up to 100% of residual isotopologues ¹H₂¹⁷O, ¹H₂¹⁸O, ¹H²H¹⁶O, ¹H²H¹⁷O, ¹H²H¹⁸O, ²H₂¹⁶O, ²H₂¹⁷O, and ²H₂¹⁸O_{.}

### Brief Description of the Drawings

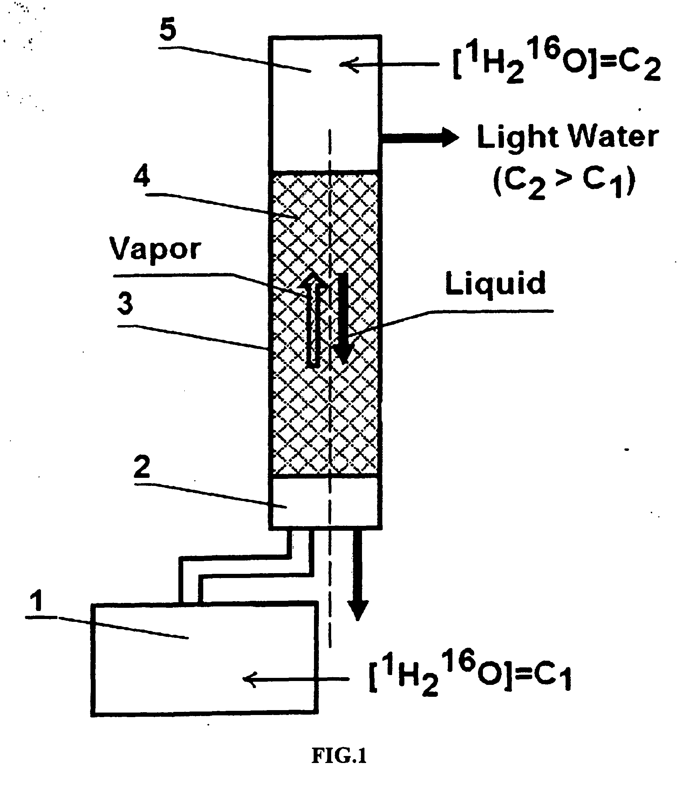

FIG.1 is a schematic side view of an apparatus for the manufacturing the water comprising from about 99.760 to about 99.999% of light isotopologue ¹H₂¹⁶O and up to 100% of residual isotopologues ¹H₂¹⁷O, ¹H₂¹⁸O, ¹H²H¹⁶O, ¹H²H¹⁷O, ¹H²H¹⁸O, ²H₂¹⁶O, ²H₂¹⁷O and ²H₂¹⁸O.

### Disclosure of Invention

The present invention provides a therapeutic composition comprising an effective amount of a biologically active agent and a pharmaceutical water, the water content of the composition being from 99.760 to 99.999% of light isotopologue ¹H₂¹⁶O and up to 100% of residual isotopologues ¹H₂¹⁷O, ¹H₂¹⁸O, ¹H²H¹⁶O, ¹H²H¹⁷O, ¹H²H¹⁸O, ²H₂¹⁶O, ²H₂¹⁷O, and ²H₂¹⁸O.

As used herein, the term "pharmaceutical water" refers to water used in pharmaceutical industry for the production of bulk drugs or manufacturing finished products (e.g. unit dosage form of drugs). Preferred pharmaceutical water of the invention is purified water (PW) and water for injection (WFI). A generally recognized compendium describing the PW and WFI grade pharmaceutical water is United States Pharmacopeia's monograph (current edition USP 23).

As used herein, the term "isotopologue" is in accordance with IUPAC Compendium of Chemical Terminology 2nd Edition (1997) and refers to a molecular entity that differs only in isotopic composition (number of isotopic substitutions), e.g. ¹H₂¹⁶O, ¹H²H¹⁶O, and ¹H₂¹⁸O.

Herein and after, term "light pharmaceutical water" refers to water comprising from 99.760 to 99.999% of light isotopologue ¹H₂¹⁶O and up to 100% of residual isotopologues ¹H₂¹⁷O, ¹H₂¹⁸O, ¹H²H¹⁶O, ¹H²H¹⁷O, ¹H²H¹⁸O, ²H₂¹⁶O, ²H₂¹⁷O, and ²H₂¹⁸O. Relative amounts of particular heavy isotopologues could vary depending upon the procedure of the preparing the water of the invention, but the sum of residual heavy isotopologues formed by heavy isotopes should not exceed 0.001 to 0.240%. Accordingly, the residual amounts of heavy isotopes in the water of the invention could vary from 0.01 ppm to 155 ppm for ²H, 1 to 360 ppm for ¹⁷O, and 1 to 2000 ppm for ¹⁸O, but the sum of heavy isotopologues formed by these residual heavy isotopes should not exceed 0.001 to 0.240%.

A method of producing the pharmaceutical water comprises a step of depleting raw water of heavy isotopologues ¹H₂¹⁷O, ¹H₂¹⁸O, ¹H²H¹⁶O, ¹H²H¹⁷O, ¹H²H¹⁸O, ²H₂¹⁶O, ²H₂¹⁷O, and ²H₂¹⁸O.

The depleting raw water of heavy isotopologues ¹H₂¹⁷O, ¹H₂¹⁸O, ¹H²H¹⁶O, ¹H₂H¹⁷O, ¹H²H¹⁸O, ²H₂¹⁶O, ²H₂¹⁷O, and ²H₂¹⁸O can be made by methods known to those skilled in the art. Preferred method for the depleting the raw water is a highly-effective distillation. The product of such depleting is light water comprising from 99.760% to 99.999% of light isotopologue ¹H₂¹⁶O and up to 100% of residual isotopologues ¹H₂¹⁷O, ¹H₂¹⁸O, ¹H²H¹⁶O, ¹H²H¹⁷O, ¹H²H¹⁸O, ²H₂¹⁶O, ²H₂¹⁷O, and ²H₂¹⁸O. The light pharmaceutical water is prepared from such light water by the methods well-known to those skilled in the art, which methods comprise steps of treating the light water such as pre-treatment, (e.g. primary filtration, coagulation and flocculation, desalination, and softening), disinfection, reverse osmosis or deionization, distillation or ultra-filtration, and chemical and microbial testing of a water sample. In accordance with the USP monograph (USP 23), purified water (PW) is obtained by distillation, ion-exchange treatment, reverse osmosis, or other suitable process and water for injection (WFI) is obtained by distillation or reverse osmosis. In practicing the invention, the sample of light pharmaceutical water should meet requirements of a national pharmacopeia's standards, for example, the US Parmacopeia's standards.

The present invention provides a therapeutic composition comprising an effective amount of a biologically active agent and a pharmaceutical water, the water content of the composition being from 99.760 to 99.999% of light isotopologue ¹H₂¹⁶O and up to 100% of residual isotopologues ¹H₂¹⁷O, ¹H₂¹⁸O, ¹H²H¹⁶O, ¹H²H¹⁷O, ¹H²H¹⁸O, ²H₂¹⁶O, ²H₂¹⁷O, and ²H₂¹⁸O.

The present invention can be used in a method of administering a biologically active agent to a mammal in need thereof, which method comprises a step of administering to said mammal the therapeutic composition comprising an effective amount of a biologically active agent and the pharmaceutical water, which water comprises from 99.760 to 99.999% of light isotopologue ¹H₂¹⁶O and up to 100% of residual isotopologues ¹H₂¹⁷O, ¹H₂¹⁸O, ¹H²H¹⁶O, ¹H²H¹⁷O, ¹H²H¹⁸O, ²H₂¹⁶O, ²H₂¹⁷O, and ²H₂¹⁸O.

In the composition of the invention, the biologically active agent is selected from the group consisting of physiologically active peptides, physiologically active proteins, and drugs selected from the group consisting of analgesic agents, anesthetic agents, antiacne agents, anti-aging agents, antiallergic agents, antianemic agents, antianginal agents, antianxiety agents, antiarrythmic agents, antiasthmatic agents, antibacterial agents, anticancer agents, anticholinergic agents, anticoagulant agents, anticonvulsant agents, antidepressant agents, antidiabetic agents, antidiarrheal agents, antidiuretic agents, antidotes, antidyskinetic agents, antidysmenorrheal agents, antiemetic agents, antifibrinolytic agents, antifibrotic agents, antifungal agents, antiglaucoma agents, antihemorrhagic agents, antihistaminic agents, antihypercalcemic agents, antyhyperglycemic agents, antihyperlipidemic agents, antihypertensive agents, antihyperthyroid agents, antihyperuricemic agents, antihypocalcemic agents, antihypoglycemic agents, antihypotensive agents, anti-inflammatory agents, anti-Kawasaki disease agents, antimalarial agents, antimethemoglobinemic agents, antimicrobial agents, antimigraine agents, antimyastenic agents, antineoplastic agents, antineuralgic agents, antineutropenic agents, antipanic agents, antipolyneuropathy agents, antiprotozoal agents, antipsoriatic agents, antipsychotic agents, antipyretic agents, antirheumatic agents, antiseborrheic agents, antispasmodic agents, auntithrombotic agents, antitremor agents, antiulcer agents, antivertigo agents, antiviral agents, anti-wrinkling agents, appetite stimulants, appetite suppressants, asthma prophylactic agents, bone resorption inhibitors, bronchodilators, contraceptive agents, corticosteroids, depigmentation agents, disinfecting agents, diuretic agents, components of dialysis solution, eastrogens, hair growth promoting agents, hair growth inhibitor agents, hemapoietic stimulants, hepatitis treatment agents, histamine H2-receptor antagonists, hormones, insulin resistance treatment agents, interferon resistance treatment agents, immunosuppressants, impotence therapy agents, keratolytic agents, miotic agents, mucolytic agents, mydriatic agents, myocardial infarction therapy or prophylactic agents, neuromuscular blocking agents, osteoporosis prophylactic or therapy agents, sedative agents, skeletal muscle relaxant, skin lightening agents, sympathomimetic agent, thrombolitic agents, vaccines, vasodilators, wound healing promoters, counterirritants, herbal extracts, retinoids, and bioflavonoids.

Nonexclusive examples of analgesic agent include acetaminophen, hydrocodone bitartrate, codeine, caffeine, acetylsalicylic acid, diclofenac, or mixture thereof.

Nonexclusive examples of anesthetic agent include lidocaine, tetracaine, and epinephrine.

Nonexclusive examples of antiacne agent include erythromycine, tetracycline, benzoyl peroxide, nicotinamide, resorcinol, doxycicline, and salycilic acid.

Nonexclusive examples of antianemic agent include cyanocobalamine, epoietin, ferrous fumarate, ferrous sulfate, ferrous citrate, and iron dextran.

Nonexclusive examples of antianginal agent include atenolol, isosorbide mononitrate, nitroglycerine, propranolol, timolol, metoprolol, and verapamil.

Nonexclusive examples of antianxiety agent include alprazolam, bromazepam, lorazepam, and diazepam.

Nonexclusive examples of antiarrythmic agent include acebutolol, atenolol, atropine, and metoprolol.

Nonexclusive examples of antiasthmatic agent include dexmethasone, and loratadine.

Nonexclusive examples of antibacterial agent include aminosalycilic acid, isoniazide, erythromycine, gentamicin, ciprofloxacin, amoxicillin, cephaxelin, and streptomycin.

Nonexclusive examples of anticancer agent include daunorubicin.

Nonexclusive examples of anticholinergic agent include atropine.

Nonexclusive examples of anticoagulant agent include heparin, warfarin, and dicumarol.

Nonexclusive examples of anticonvulsant agent include clonazepam, diazepam, and valproic acid.

Nonexclusive examples of antidepressant agent include amitriptyline, and fluoxetin.

Nonexclusive examples of antidiabetic agent include insulin, metformin, succinic acid, and sulfonylureas like as glyburide.

Nonexclusive examples of antidiarrheal agent include codeine, and bismuth subsalycilate.

Nonexclusive examples of antidiuretic agent include vasopressin.

Nonexclusive examples of antidote include glucagon to calcium channel blocking agents, and penicillamine to heavy metals.

Nonexclusive examples of antidyskinetic agent include levodopa.

Nonexclusive examples of antidysmenorrheal agent include diclofenac.

Nonexclusive examples of antiemetic agent include chlorpromazine.

Nonexclusive examples of antifibrinolytic agent include aminocaproic acid.

Nonexclusive examples of antifibrotic agent include potassium aminobenzoate.

Nonexclusive examples of antifungal agent include ketoconazol.

Nonexclusive examples of antiglaucoma agent include carbachol, and pilocarpine.

Nonexclusive examples of antihemorrhagic agent include factor IX, and aminocaproic acid.

Nonexclusive examples of antihistaminic agent include ranitidine hydrochloride, and loratadine.

Nonexclusive examples of antihypercalcemic agent include calcitonin, etidronate and other biphosphonates.

Nonexclusive examples of antyhyperglycemic agent include metformin.

Nonexclusive examples of antihyperlipidemic agent include clofibrate, and fluvastatin.

Nonexclusive examples of antihypertensive agent include atenolol, captopril, lisinopril, and verapamil.

Nonexclusive examples of antihyperthyroid agent include potassium iodide.

Nonexclusive examples of antihyperuricemic agent include allopurinol.

Nonexclusive examples of antihypocalcemic agent include calcium acetate.

Nonexclusive examples of antihypoglycemic agent include glucagon.

Nonexclusive examples of antihypotensive agent include dihydroergotamine.

Nonexclusive examples of anti-inflammatory agent include acetylsalycilic acid, choline salycilate, ibuprofen, diclofenac, indomethacin, and dexamethasone.

Nonexclusive examples of anti-Kawasaki disease agent include immune globulin.

Nonexclusive examples of antimalarial agent include quanidine.

Nonexclusive examples of antimethemoglobinemic agent include methylene blue.

Nonexclusive examples of antimyastenic agent include neostygmine.

Nonexclusive examples of antineoplastic agent include asparaginase, carboplatin, daunorubicin, interferon, tamoxifen, and fluorouracil.

Nonexclusive examples of antineuralgic agent include carbamazepine.

Nonexclusive examples of antipanic agent include clonazepam.

Nonexclusive examples of antipolyneuropathy agent include immune globulin.

Nonexclusive examples of antiprotozoal agent include chloroquine, and furazolidone.

Nonexclusive examples of antipsoriatic agent include methotrexate.

Nonexclusive examples of antipsychotic agent include carbamazepine.

Nonexclusive examples of antipyretic agent include acetaminophen.

Nonexclusive examples of antirheumatic agent include methotrexate.

Nonexclusive examples of antiseborrheic agent include pyrithione, salycilic acid.

Nonexclusive examples of antispasmodic agent include glucagon.

Nonexclusive examples of antithrombotic agent include acetylsalycilic acid.

Nonexclusive examples of antitremor agent include diazepam.

Nonexclusive examples of antiulcer agent include omeprazole.

Nonexclusive examples of antivertigo agent include diphenidol.

Nonexclusive examples of antiviral agent include acyclovir, and ribavirin.

Nonexclusive examples of appetite stimulant include dronabinol.

Nonexclusive examples of appetite suppressant include phentermine.

Nonexclusive examples of asthma prophylactic agent include theophylline.

Nonexclusive examples of bone resorption inhibitor include calcitonin.

Nonexclusive examples of bronchodilator include theophylline, and albuterol.

Nonexclusive examples of contraceptive agent include levonorgestrel.

Nonexclusive examples of corticosteroid include dexamethasone.

Nonexclusive examples of diuretic agent include furosemide, and hydrochlorothiazide.

Nonexclusive examples of eastrogen include estradiol.

Nonexclusive examples of hemapoietic stimulant include epoietin.

Nonexclusive examples of hepatitis treatment agent include interferon alpha.

Nonexclusive examples of histamine H2-receptor antagonist include cimetidine.

Nonexclusive examples of hormones include melatonin, thyroid hormones, thyroxine, triiodothyronine, adrenaline, noradrenaline, glucocorticoids (e.g. cortisol), mineralocorticoids (e.g. aldosteron), estrogens (e.g. estradiol), progesterone, androgens (e.g. testosteron), calcitriol, and calciferol.

Nonexclusive examples of insulin resistance treatment agents or interferon resistance treatment agents include succinic acid or salts thereof.

Nonexclusive examples of immunosuppressant include cyclosporine.

Nonexclusive examples of impotence therapy agent include papaverine.

Nonexclusive examples of keratolytic agent include benzoyl peroxide.

Nonexclusive examples of miotic agent include carbachol.

Nonexclusive examples of mucolytic agent include acetylcysteine.

Nonexclusive examples of mydriatic agent include atropine.

Nonexclusive examples of myocardial infarction therapy or prophylactic agent include acetylsalycilic acid.

Nonexclusive examples of neuromuscular blocking agent include succinylcholine.

Nonexclusive examples of osteoporosis prophylactic or therapy agent include estrogens.

Nonexclusive examples of sedative agent include diazepam.

Nonexclusive examples of skeletal muscle relaxant include phenytoin.

Nonexclusive examples of thrombolitic agent include urokinase.

Nonexclusive examples of vasodilator include enalapril.

The drug is generally used individually at levels from 0.0005% to 50.0%, preferably from 0.005% to 5.0% by weight of the composition.

Preferably, the physiologically active peptide- or protein is selected from the group consisting of cytokines, peptide hormones, growth factors, factors acting on the cardiovascular system, factors acting on the central and peripheral nervous systems, factors acting on humoral electrolytes, hematopoietic factors, factors acting on bone and skeleton, factors acting on the gastrointestinal system, factors acting on the immune system, factors acting on the genital organs, and enzymes.

Nonexclusive examples of cytokines include interferons (e.g. interferon-alpha, -beta, and -gamma) and interleukins (e.g. interleukin 2 through 12).

Nonexclusive examples of protein or peptide hormones include insulin, insulin-like growth factor-1, growth hormone, luteinizing hormone-releasing hormone (LH-RH), adrenocorticotropic hormone (ACTH), amylin, oxytocin, activin, amylin, angiotensin, atrial natriuretic peptides (ANP), calcitonin, cholecystokinin (CCK), ciliary neurotrophic factor (CNTF), corticotropin-releasing hormone (CRH or CRF), erythropoietin, follicle-stimulating hormone (FSH), gastrin, gastrin inhibitory peptide (GIP), gastrin-releasing peptide, ghrelin, glucogon, gonadotropin-releasing factor (GnRF or GNRH), growth hormone releasing hormone (GRF, GRH), human chorionic gonadotropin (hCH), inhibin A, inhibin B, leptin, lipotropin (LPH), luteinizing hormone (LH), motilin, neuropeptide Y, oxytocin, pancreatic polypeptide, parathyroid hormone (PTH), placental lactogen, prolactin (PRL), prolactin-release inhibiting factor (PIF), prolactin-releasing factor (PRF), PYY3-36, secretin, somatostatin (SIF, growth hormone-release inhibiting factor, GIF), thrombopoietin, thyrotropin (thyroid-stimulating hormone, TSH), thyrotropin-releasing factor (TRH or TRF), vasoactive intestinal peptide (VIP), vasopressin (antidiuretic hormone, ADH) and combinations thereof.

Nonexclusive examples of growth factors include nerve growth factors (NGF, NGF-2/NT-3), epidermal growth factor (EGF), fibroblast growth factor (FGF), insulin-like growth factor (IGF), transforming growth factor (IGF), platelet-derived cell growth factor (PDGF), keratinocyte growth factor (TGF), and hepatocyte growth factor (HGF).

Nonexclusive examples of factors acting on the cardiovascular system include which control blood pressure, arteriosclerosis, etc., such as endothelins, endothelin inhibitors, endothelin antagonists, vasoppressin, renin, angiotensin I, angiotensin II, angiotensin III, angiotensin I inhibitor, angiotensin II receptor antagonist, atrial naturiuretic peptide (ANP), and antianythmic peptide.

Nonexclusive examples of factors acting on the central and peripheral nervous systems include opioid peptides (e.g. enkepharins, endorphins, kyotorphins), neurotrophic factor (NTF), calcitonin gene-related peptide (CGRP), thyroid hormone releasing hormone (TRH), glial-derived neurotrophic factor (GDNF), brain-derived neurotrophic factor (BDNF), and neurotrophines (e.g. NT3, NT4).

Nonexclusive examples of factors acting on humoral electrolytes include calcitonin.

Nonexclusive examples of hematopoietic factors include erythropoietin, granulocyte colony stimulating factor (G-CSF), granulocyte-macrophage stimulating factor (GM-CSF) and macrophage colony stimulating factor (M-CSF), and thrombopoietin.

Nonexclusive examples of factors acting on the gastrointestinal system include secretin and gastrin.

Nonexclusive examples of factors acting on the immune system include interferons (e.g. interferon-alpha, and -gamma), monoclonal antibody, and naturally occurring, chemically synthesized or recombinant peptides or proteins which may act as antigens. These factors are administered, either independently, as coupled to haptens, or together with an adjuvant, in the formulations according to the present invention.

Nonexclusive examples of factors acting on bone and skeleton include parathyroid hormone and calcitonin.

Nonexclusive examples of factors acting on the genital organs include luteinizing hormone-releasing hormone (LH-RH) and luteinizing hormone.

Nonexclusive examples of enzymes include superoxide dismutase (SOD), urokinase, asparaginase, and kallikreins.

These peptides or proteins may further include glycosylated peptides or proteins, and chemically modified peptides or proteins. Nonexclusive examples of chemically modified peptides or proteins include peptides or proteins linked by a covalent chemical bond to synthetic polymers such as polyethylene glycol, natural polymers such as chondroitin, polysaccharides, etc. and those chemically modified with non-peptide substances. The non-peptide substances mentioned above may be ligands for receptors or antigens for antibodies.

Furthermore, the peptide or protein may be a plurality of peptides joined together by chemical means or by genetic engineering technology.

Typically, the peptide or protein is presented in the composition of the invention in amounts close to its normal dosage and daily regimen ranges as detailed in medical literature for injective dosage forms. Preferably, the peptide or protein is generally used individually at levels from 0.00001 % to 5.0%, preferably from 0.0001% to 1% by weight of the composition.

Preferably, the amount of the pharmaceutical water in the composition of the invention is from 1.0 to 99.9% based upon the total weight of the composition. More preferably, from 50.0 to 99.9% based upon the total weight of the composition.

The composition of the invention is provided in the pH range which does not remarkably affect the activity of the physiologically active peptide or protein and is physiologically acceptable. The preferred range is pH 2 to pH 10. The more preferred range is 3 to pH 9, and the most desirable range is pH 3.5 to pH 8. Preferably, buffer include, but are not limited to, glycine, citrate, succinate, fumarate, malate, or phosphate buffer.

The compositions of the invention are prepared by known procedures using well-known ingredients. In making the compositions, the biologically active agent will usually be mixed with the light water and diluted by appropriate diluent or carrier. Additively, the composition of the invention can comprise preservatives, flavors, colorants, and etc. Additively, the composition of the present invention may be further supplemented with an absorption promoter which assists in the absorption of the pharmacologically active substance. The absorption promoter may be any promoter that is pharmaceutically acceptable. Thus, there can be sodium salicylate and salicylic acid derivatives (acetyl salicylate, choline salicylate, salicylamide, etc.), amino acids and salts thereof (e.g. monoaminocarboxlic acids such as glycine, alanine, phenylalanine, proline, hydroxyproline, etc., hydroxyamino acids such as serine etc., acidic amino acids such as aspartic acid, glutamic acid, etc. and basic amino acids such as lysine etc., inclusive of their alkali metal or alkaline earth metal salts), N-acetylamino acids (N-acetylalanine, N-acetylphenylalanine, N-acetylserine, N-acetylglycine, N-acetyllysine, N-acetylglutamic acid, N-acetylproline, N-acetylhydroxyproline, etc.) and their salts (alkali metal salts and alkaline earth metal salts), substances which are generally used as emulsifiers (e.g. sodium oleyl phosphate, sodium lauryl phosphate, sodium lauryl sulfate, sodium myristyl sulfate, polyoxyethylene alkyl ethers, polyoxyethylene alkyl esters, etc.), caproic acid, lactic acid, malic acid and citric acid and alkali metal salts thereof, pyrrolidonecarboxylic acids, alkylpyrrolidonecarboxylic acid esters, N-alkylpyrrolidones, proline acyl esters, and etc. Such optional ingredients generally are used individually at levels from 0.0005% to 10.0%, preferably from 0.005% to 1.0% by weight of the composition.

The therapeutic composition can be formulated in a variety of unit dosage form. Such forms include, but are not limited to, gel tablets, sprays, syrups, suppositories, drops, and solutions.

The therapeutic composition in a unit dosage form can be administered by a variety of routes. Such routes include, but are not limited to, oral, parenteral, transdermal, transmucosal, intradermal, intranasal, rectal, or topical.

Oral dosage form (e.g. syrup, gel tablet) is ingested and this method can be reapplied from 1 to 5, preferably from 1 to 3 times per day.

Transmucosal dosage form (e.g. sublingual spray, gel tablet, or solution) is applied on sublingual mucosa of the mammal for period at least 10 second and this method can be reapplied from 1 to 5, preferably from 1 to 3 times per day.

Parenteral dosage form (e.g. solution for injections) is injected to the mammal (e.g subcutaneously or intramuscularly) and this method can be reapplied.

Nasal dosage form (e.g. nasal spray or drops) is applied on nasal mucosa of the mammal and this method can be reapplied from 1 to 5, preferably from 1 to 3 times per day.

Topical dosage form (e.g. gel) is applied on skin of the mammal, and is preferably left on the skin for a period of at least 15 minutes, more preferably at least 30 minutes, even more preferably at least 1 hour, most preferably for at least several hours, e.g., up to 12 hours. This method can be reapplied from 1 to 5, preferably from 1 to 3 times per day.

The following examples are presented to demonstrate the invention. The examples are illustrative only and are not intended to limit the scope of the invention in any way.

### Example 1

This example demonstrates the method for producing pharmaceutical water of the invention.

Raw light water comprising 99.99% of light isotopologue ¹H₂¹⁶O and up to 100% of residual isotopologues ¹H₂¹⁷O, ¹H₂¹⁸O, ¹H²H¹⁶O, ¹H²H¹⁷O, ¹H²H¹⁸O, ²H₂¹⁶O, ²H₂¹⁷O, and ²H₂¹⁸O is prepared by highly-effective distillation of natural raw water comprising 99.73% of light isotopologue ¹H₂¹⁶O with using the apparatus of FIG.1 under temperature 60°C and pressure 0.2 bars. The process of the distillation comprises evaporating natural water comprising 99.73% (C₁) of light isotopologue ¹H₂¹⁶O in boiling means 1 to produce water vapor; supplying the water vapor to the bottom 2 of distillation column 3; carrying out vapor-liquid contact between a descending liquid and an ascending vapor mainly on the surface of the contact device 4 (e.g. structured or random packing) within the distillation column, at which time the liquid and the vapor flow in mutually opposite directions over the surface of the contact device along a main flow direction which is along a direction of the column axis; condensing water vapor with concentration of light isotopologue ¹H₂¹⁶O 99.99% (C₂) on condenser 5 installed on upper bound of the distillation column 3; and collecting a part of condensate as condensed raw light water comprising 99.99% of light isotopologue ¹H₂¹⁶O (C₂ > C₁) appropriate for producing light pharmaceutical water. Then, the raw light water is treated consequently by procedure of reverse osmosis. Resulted WFI grade pharmaceutical water comprising 99.99% of light isotopologue ¹H₂¹⁶O is tested on chemical and microbial contaminants as prescribed by national pharmacopeia's standards.

### Example 2

This example shows a representative formulation comprising insulin.

| | Content |
|---|---|
| Water for injections (99.99% of light isotopologue ¹H₂¹⁶O) | 1 ml |
| Human recombihant insulin | 3.5 mg (100 IU) |
| Sodium chloride | 5 mg |
| Polysorbate 20 | 0.01 mg |
| m-Cresol | 2.7 mg |

The method for preparing the composition described in Example 2 was as follows: the human recombinant insulin, light water for injections and other components were mixed.

### Example 3

This example shows a representative formulation comprising interferon-alpha.

| | Content |
|---|---|
| Water for injections (99.99% of light isotopologue ¹H₂¹⁶O) | 0.5 ml |
| Human recombinant interferon-alpha | 10 µg |
| Sodium chloride | 5.9 mg |
| Sodium phosphate | 3.8 mg |

Th method for preparing the composition described in Example 3 was as follows: the human recombinant interferon-alpha, light water for injections and other components were mixed.

### Example 4

This example shows a representative formulation comprising interferon-beta.

| | Content |
|---|---|
| Water for injections (99.99% of light isotopologue ¹H₂¹⁶O) | 1 ml |
| Human recombinant interferon-beta | 30 µg |
| Albumin | 15 mg |
| Sodium chloride | 5.8 mg |
| Dibasic sodium phosphate | 5.7 mg |
| Monobasic sodium phosphate | 1.2 mg |

The method for preparing the composition described in Example 4 was as follows: the human recombinant interferon-beta, light water for injections and other components were mixed.

### Example 5

This example shows a representative formulation comprising erythropoietin.

| | Content |
|---|---|
| Water for injections (99.99% of light isotopologue ¹H₂¹⁶O) | 1 ml |
| Human recombinant erythropoietin | 10,000 IU |
| Albumin | 2.5 mg |
| Sodium citrate | 5.8 mg |
| Sodium chloride | 5.8 mg |
| Citric acid | 0.06 mg |

The method for preparing the composition described in Example 5 was as follows: the human recombinant erythropoietin, light water for injections and other components were mixed.

### Example 6

This example shows a representative formulation comprising calcitonin.

| | Content |
|---|---|
| Water for injections (99.99% of light isotopologue ¹H₂¹⁶O) | 1 ml |
| Salmon calcitonin | 2200 IU |
| Sodium chloride | 8.5 mg |

The method for preparing the composition described in Example 6 was as follows: the salmon calcitonin, light water for injections and other components were mixed.

### Example 7

This example shows a representative formulation comprising G-CSF.

| | Content |
|---|---|
| Water for injections (99.99% of light isotopologue ¹H₂¹⁶O) | 1 ml |
| Human recombinant G-CSF | 300 µg |
| Acetate | 0.59 mg |
| Sodium | 0.035 mg |
| Tween 80 | 0.004% |
| Sorbitol | 50 mg |

The method for preparing the composition described in Example 7 was as follows: the human recombinant G-CSF, light water for injections and other components were mixed.

### Example 8

This example shows a representative formulation comprising GM-CSF.

| | Content |
|---|---|
| Water for injections (99.99% of light isotopologue ¹H₂¹⁶O) | 1 ml |
| Human recombinant GM-CSF | 500 µg |
| Sodium citrate | 5.8 mg |
| Sodium chloride | 5.8 mg |
| Citric acid | 0.06 mg |

The method for preparing the composition described in Example 8 was as follows: the human recombinant GM-CSF, light water for injections and other components were mixed.

### Example 9

This example shows a representative formulation comprising GDNF.

| | Content |
|---|---|
| Water for injections (99.99% of light isotopologue ¹H₂¹⁶O) | 1 ml |
| Human recombinant GDNF | 500 µg |
| Sodium citrate | 5.8 mg |
| Sodium chloride | 5.8 mg |
| Citric acrid | 0.06 mg |

The method for preparing the composition described in Example 9 was as follows: the human recombinant GDNF, light water for injections and other components were mixed.

### Example 10

This example shows a representative formulation comprising growth hormone.

| | Content |
|---|---|
| Water for injections (99.99% of light isotopologue ¹H₂¹⁶O) | 1.5 ml |
| Human recombinant growth hormone | 5 mg |
| Sodium citrate | 5.8 mg |
| Sodium chloride | 5.8 mg |
| Citric acid | 0.06 mg |

The method for preparing the composition described in Example 10 was as follows: the human recombinant growth hormone, light water for injections and other components were mixed.

### Example 11

This example shows a representative formulation comprising IL-1 receptor antagonist.

| | Content |
|---|---|
| Water for injections (99.99% of light isotopologue ¹H₂¹⁶O) | 1 ml |
| Human recombinant IL-1 receptor antagonist | 100 mg |
| Sodium citrate | 1.29 mg |
| Sodium chloride | 5.48 mg |
| Polysorbate 80 | 0.70 mg |

The method for preparing the composition described in Example 11 was as follows: the human recombinant IL-1 receptor antagonist, light water for injections and other components were mixed.

### Example 12

This example shows a representative formulation comprising anti-VEGF monoclonal antibody.

| | Content |
|---|---|
| Water for injections (99.99% of light isotopologue ¹H₂¹⁶O) | 1 ml |
| anti-VEGF monoclonal antibody | 25 mg |
| Trehalose | 60 mg |
| Sodium phosphate (monobasic) | 5.8 mg |
| Sodium phosphate (dibasic) | 1.2 mg |
| Sodium chloride | 5.48 mg |
| Polysorbate 20 | 0.40 mg |

The method for preparing the composition described in Example 12 was as follows: anti-VEGF monoclonal antibody, light water for injections and other components were mixed.

### Example 13

This example shows a representative formulation comprising influenza virus vaccine.

| | Content |
|---|---|
| Water for injections (99.99% of light isotopologue ¹H₂¹⁶O) | 1 ml |
| Viral antigen (hemagglutinin) | 90 µg |

The method for preparing the composition described in Example 13 was as follows viral antigen (hemagglutinin), light water for injections and other components were mixed.

### Example 14

This example shows a representative formulation comprising artificial tear (ophtalmological agent).

| | Content |
|---|---|
| Water for injections (99.99% of light isotopologue ¹H₂¹⁶O) | 1 ml |
| Polyacrylic acid | 2 mg |
| Sorbitol | 40 mg |
| Benzalkonium chloride | 2 mg |

The method for preparing the composition described in Example 14 was as follows: polyacrylic acid, sorbitol, benzalkonium chloride, and light water for injections were mixed.

### Example 15

This example shows representative formulation comprising dextran 70 (blood substitute).

| | Content |
|---|---|
| Water for injections (99.99% of light isotopologue ¹H₂¹⁶O) | 1 ml |
| Dextran 70 | 60 mg |
| Sodium chloride | 9 mg |

The method for preparing the composition described in Example 15 was as follows: dextran 70, sodium chloride, and light water for injections were mixed.

### Example 16

This example shows a representative formulation comprising naloxone (antidote).

| | Content |
|---|---|
| Water for injections (99.99% of light isotopologue ¹H₂¹⁶O) | 1 ml |
| Naloxone hydrochloride | 0.4 mg |

The method for preparing the composition described in Example 16 was as follows: naloxone hydrochloride and light water for injections were mixed.

### Example 17

This example shows a representative formulation comprising sodium chloride (agent correcting electrolyte balance).

| | Content |
|---|---|
| Water for injections (99.99% of light isotopologue ¹H₂¹⁶O) | 1 ml |
| Sodium chloride | 9 mg |

The method for preparing the composition described in Example 17 was as follows: sodium chloride and light water for injections were mixed.

### Example 18

This example shows a representative dermatological formulation for skin hydration.

| | Content |
|---|---|
| Purified Water (99.99% of light isotopologue ¹H₂¹⁶O) | 21 g |
| Glycerin | 5g |
| Methylhydroxypropylcellulose | 2.5 g |

The method for preparing the composition described in Example 18 was as follows: glycerin, purified light water and methylhydroxypropylcellulose were mixed.

### Example 19

This example shows a representative formulation comprising interleukin-2.

| | Content |
|---|---|
| Water for injections (99.99% of light isotopologue ¹H₂¹⁶O) | 1.2 ml |
| Human recombinant interleukin-2 | 18x10⁶IU |
| Mannitol | 50 mg |
| Sodium dodecylsulfate | 0.18 mg |
| Dibasic sodium phosphate | 0.89 mg |
| Monobasic sodium phosphate | 0.17 mg |

The method for preparing the composition described in Example 19 was as follows: the human recombinant interleukin-2, light water for injections and other components were mixed.

### Example 20

This example demonstrates an advantageous efficacy of interferon delivery through administration of interferon composition made on light pharmaceutical water vs. pharmaceutical water.

Material. Human recombinant interferon-alpha (IFN) was used.

Treatment. Male C57B1 mice about 20 g weights were administered oromucosally with 170 ng/mouse IFN in form of 20 µl/mouse solution made on natural distilled water (99.73% of light isotopologue ¹H₂¹⁶O, control) or light pharmaceutical water (99.90% of light isotopologue ¹H₂¹⁶O) in citrate buffer under pH 5.4-5.5. Plasma IFN levels were measured at 7 min after the administration with immunoenzyme assay specific to human IFN. Data are presented in table below as plasma IFN mean ±SD (n=6) in ng/ml.

| Treatment | Plasma IFN, ng/ml |
|---|---|
| IFN plus pharmaceutical water (control) | 3.7 ± 2.0 |
| IFN plus light pharmaceutical water | 15.0 ± 1.8* |

| | |
|---|---|
| *Differs significantly of control (p<0.0001) | |

### Example 21

This example demonstrates an advantageous efficacy of interferon delivery through administration of interferon composition made on light pharmaceutical water vs. pharmaceutical water.

Material. Human recombinant interferon-alpha (IFN) was used.

Treatment. Male C57B1 mice about 20 g weights were depilated 72 hours before the experiment. 100 ng/mouse IFN in form of 100 µl/mouse solution made on natural distilled water (99.73% of light isotopologue ¹H₂¹⁶O, control) or light pharmaceutical water (99.90% of light isotopologue ¹H₂¹⁶O) in succinate buffer under pH 5.1 were applied on skin depilated area of about 1 cm². Plasma IFN levels were measured at 10 min with immunoenzyme assay specific to human IFN. Data are presented in table below as plasma IFN mean ± SD (n=5) in ng/ml.

| Treatment | Plasma IFN, ng/ml |
|---|---|
| IFN plus pharmaceutical water (control) | 0.37 ± 0.09 |
| IFN plus light pharmaceutical water | 3.62 ± 1.01* |

| | |
|---|---|
| *Differs significantly of control (p<0.0001) | |

### Example 22

This example shows a representative formulation for a sublingual spray comprising interferon alpha.

| | Content, weight % |
|---|---|
| Light pharmaceutical water (99.99% of light isotopologue ¹H₂¹⁶O) | 92.00 |
| Human recombinant Interferon alpha2a | 0.85 |
| Glycerol | 5 |
| Succinic acid | 1.2 |
| Sodium hydroxide | 0.95 |

The method for preparing the composition described in Example 22 was as follows: the above-mentioned ingredients were mixed in the conventional -manner to prepare the sublingual spray comprising interferon alpha.

### Example 23

This example shows a representative formulation for a sublingual spray comprising insulin.

| | Content, weight % |
|---|---|
| Light pharmaceutical water (99.99% of light isotopologue ¹H₂¹⁶O) | 92.24 |
| Human recombinant insulin | 0.81 |
| Glycerol | 5 |
| Succinic acid | 1.0 |
| Sodium hydroxide | 0.95 |

The method for preparing the composition described in Example 23 was as follows: the above-mentioned ingredients were mixed in the conventional manner to prepare the sublingual spray comprising insulin.

### Example 24

This example shows a representative formulation for a sublingual spray comprising erythropoietin.

| | Content, weight % |
|---|---|
| Light pharmaceutical water (99.99% of light isotopologue ¹H₂¹⁶O) | 92.25 |
| Human recombinant erythropoietin | 0.80 |
| Glycerol | 5 |
| Citric acid | 1.0 |
| Sodium hydroxide | 0.95 |

The method for preparing the composition described in Example 24 was as follows: the above-mentioned ingredients were mixed in the conventional manner to prepare the sublingual spray comprising erythropoietin.

### Example 25

This example shows a representative formulation for a sublingual spray comprising calcitonin.

| | Content, weight % |
|---|---|
| Light pharmaceutical water (99.99% of light isotopologue ¹H₂¹⁶O) | 92.25 |
| Calcitonin | 0.80 |
| Glycerol | 5 |
| Succinic acid | 1.0 |
| Sodium hydroxide | 0.95 |

The method for preparing the composition described in Example 25 was as follows: the above-mentioned ingredients were mixed in the conventional manner to prepare the sublingual spray comprising calcitonin.

## Claims

1. A therapeutic composition comprising an effective amount of a biologically active agent and a pharmaceutical water, the water content of the composition being from 99.760 to 99.999% of light isotopologue ¹H₂¹⁶O and up to 100% of residual isotopologues ¹H₂¹⁷O, ¹H₂¹⁸O, ¹H²H¹⁶O, ¹H²H¹⁷O, ¹H²H¹⁸O, ²H₂¹⁶O, ²H₂¹⁷O, and ²H₂¹⁸O,
wherein the biologically active agent is selected from the group consisting of physiologically active peptides, physiologically active proteins, and drugs selected from the group consisting of analgesic agents, anesthetic agents, antiacne agents, anti-aging agents, antiallergic agents, antianemic agents, antianginal agents, antianxiety agents, antiarrythmic agents, antiasthmatic agents, antibacterial agents, anticancer agents, anticholinergic agents, anticoagulant agents, anticonvulsant agents, antidepressant agents, antidiabetic agents, antidiarrheal agents, antidiuretic agents, antidotes, antidyskinetic agents, antidysmenorrheal agents, antiemetic agents, antifibrinolytic agents, antifibrotic agents, antifungal agents, antiglaucoma agents, antihemorrhagic agents, antihistaminic agents, antihypercalcemic agents, antyhyperglycemic agents, antihyperlipidemic agents, antihypertensive agents, antihyperthyroid agents, antihyperuricemic agents, antihypocalcemic agents, antihypoglycemic agents, antihypotensive agents, anti-inflammatory agents, anti-Kawasaki disease agents, antimalarial agents, antimethemoglobinemic agents, antimicrobial agents, antimigraine agents, antimyastenic agents, antineoplastic agents, antineuralgic agents, agents, antiprotozoal agents, antipsoriatic agents, antipsychotic agents, antipyretic agents, antirheumatic agents, antiseborrheic agents, antispasmodic agents, antithrombotic agents, antitremor agents, antiulcer agents, antivertigo agents, antiviral agents, anti-wrinkling agents, appetite stimulants, appetite suppressants, asthma prophylactic agents, bone resorption inhibitors, bronchodilators, contraceptive agents, corticosteroids, depigmentation agents, disinfecting agents, diuretic agents, components of dialysis solution, eastrogens, hair growth promoting agents, hair growth inhibitor agents, hemapoietic stimulants, hepatitis treatment agents, histamine H2-receptor antagonists, hormones, insulin resistance treatment agents, interferon resistance treatment agents, immunosuppressants, impotence therapy agents, keratolytic agents, miotic agents, mucolytic agents, mydriatic agents, myocardial infarction therapy or prophylactic agents, neuromuscular blocking agents, osteoporosis prophylactic or therapy agents, sedative agents, skeletal muscle relaxant, skin lightening agents, sympathomimetic agent, thrombolitic agents, vaccines, vasodilators, wound healing promoters, counterirritants, herbal extracts, retinoids, and bioflavonoids.

2. The composition according to claim 1, wherein the physiologically active peptide or protein is selected from the group consisting of cytokines, peptide hormones, growth factors, factors acting on the cardiovascular system, factors acting on the central and peripheral nervous systems, factors acting on humoral electrolytes, hematopoietic factors, factors acting on bone and skeleton, factors acting on the gastrointestinal system, factors acting on the immune system, factors acting on the genital organs, and enzymes.

3. The composition according to claim 2, wherein the cytokine is interferon alpha.

4. The composition according to claim 2, wherein the peptide hormone is insulin.

5. The composition according to claim 2, wherein the growth factor is insulin-like growth factor.

6. The composition according to claim 2, wherein the peptide hormone is calcitonin.

7. The composition according to claim 2, wherein the peptide hormone is growth hormone.

8. The composition according to claim 2, wherein the hematopoietic factor is erythropoietin.

9. The composition according to claim 2, wherein the factor acting on the immune system is vaccine.

10. The composition according to claim 1, wherein the amount of the pharmaceutical water is from 1.0 to 99.9% based upon the total weight of the composition.

11. The composition according to claim 10, wherein the amount of the pharmaceutical water is from 50.0 to 99.9% based upon the total weight of the composition.

## Patentansprüche

1. Therapeutische Zusammensetzung, die eine wirksame Menge eines biologisch wirksamen Stoffes und pharmazeutisches Wasser enthält, wobei der Wassergehalt der Zusammensetzung ist: 99,760 bis 99,999 % des leichten Isotopologs ¹H₂¹⁶O und auf 100 % weitere Isotopologe ¹H₂¹⁷O, ¹H₂¹⁸O, ¹H²H¹⁶O, ¹H²H¹⁷O, ¹H²H¹⁸O, ¹H₂¹⁶O, 2H₂¹⁶O, ²H₂¹⁷O und ²H₂¹⁸O, und
wobei der biologisch wirksam Stoff ausgewählt ist unter: physiologisch aktiven Peptiden, physiologisch aktiven Proteinen und Arzneimitteln, die unter Analgetika, Anästhetika, Anti-Akne-Wirkstoffen, Anti-Aging-Wirkstoffen, Antiallergika, Antianämika, antianginösen Wirkstoffen, Anxiolytika, Antiarrhythmika, Antiasthmatika, antibakteriellen Wirkstoffen, Antikrebsmitteln, Anticholinergika, Antikoagulantien, Antikonvulsiva, Antidepressiva, Antidiabetika, Antidiarrhoika, antidiuretischen Wirkstoffen, Gegengiften, antidyskinetischen Wirkstoffen, Antidysmenorrhoika, Antiemetika, Antifibrinolytika, Antifibrotika, antimykotischen Wirkstoffen, Glaukommittel, Antihämorrhagika, Antihistaminika, antihypercalcämischen Wirkstoffen, antihyperglykämischen Wirkstoffen, antihyperlipidämischen Wirkstoffen, blutdrucksenkenden Wirkstoffen, Anti-Hyperthyreose-Wirkstoffen, Anti-Hyperurikämie-Wirkstoffen, antihypocalcämischen Wirkstoffen, antihypoglykämischen Wirkstoffen, Wirkstoffen gegen Hypotonie, entzündungshemmenden Wirkstoffen, Anti-Kawasaki-Syndrom-Wirkstoffen, Antimalariamitteln, Anti-Methämoglobinämie-Wirkstoffen, antimikrobiellen Wirkstoffen, Antimigränemitteln, Anti-Myasthenia-Wirkstoffen, antineoplastischen Wirkstoffen, Antineuralgika, Anti-Neutropenie-Wirkstoffen, Mitteln gegen Panik, Anti-Polyneuropathie-Wirkstoffen, Antiprotozoenmittel, Antipsoriatika, Antipsychotika, Antipyretika, Antirheumatika, Antiseborrhoika, krampflösenden Wirkstoffen, Antithrombotika, AntiTremor-Wirkstoffen, Wirkstoffen gegen Ulcera, Anti-Vertigo-Wirkstoffen, antiviralen Wirkstoffen, Wirkstoffen gegen Falten, Appetitanregern, Appetitzüglern, Asthma-vorbeugenden Wirkstoffen, Hemmstoffen der Knochenresorption, Bronchodilatatoren, Kontrazeptiva, Corticosteroiden, Depigmentierungsmitteln, Desinfektionsmitteln, Diuretika, Komponenten von Dialyselösungen, Estrogenen, haarwachstumsfördernden Stoffen, das Haarwachstum hemmenden Stoffen, hämatopoetischen Stoffen, Wirkstoffen zur Behandlung von Hepatitis, Histamin-H2-Rezeptor-Antagonisten, Hormonen, Wirkstoffen zur Behandlung von Insulinresistenz, Wirkstoffen zur Behandlung von Interferonresistenz, Immunsuppressiva, Wirkstoffen für die Impotenztherapie, Keratolytika, Miotika, schleimlösenden Mittel, Mydriatika, Wirkstoffen für die Therapie oder Prophylaxe von Myokard-Infarkt, neuromuskulären Blockern, Wirkstoffen für die Therapie oder Prophylaxe von Osteoporose, Sedativa, Skelettmuskelrelaxantien, Hautaufhellungsmitteln, Sympathomimetika, thrombolytischen Stoffen, Impfstoffen, Vasodilatoren, wundheilungsfördernden Wirkstoffen, Gegenreizmitteln, Pflanzenextrakten, Retinoiden und Bioflavonoiden ausgewählt sind.

2. Zusammensetzung nach Anspruch 1, wobei das physiologisch aktive Peptid oder Protein ausgewählt ist unter: Cytokinen, Peptidhormonen, Wachstumsfaktoren, Faktoren, die auf das cardiovaskuläre System einwirken, Faktoren, die auf das zentrale und periphere Nervensystem einwirken, Faktoren, die auf humorale Elektrolyte einwirken, hämatopoetische Faktoren, Faktoren, die auf Knochen und Skelett einwirken, Faktoren, die auf den Magen-Darm-Trakt einwirken, Faktoren, die auf das Immunsystem einwirken, Faktoren, die auf die Geschlechtsorgane einwirken, und Enzymen.

3. Zusammensetzung nach Anspruch 2, wobei das Cytokin das Interferon-alpha ist.

4. Zusammensetzung nach Anspruch 2, wobei das Peptidhormon das Insulin ist.

5. Zusammensetzung nach Anspruch 2, wobei der Wachstumsfaktor der insulinähnliche Wachstumsfaktor ist.

6. Zusammensetzung nach Anspruch 2, wobei das Peptidhormon das Calcitonin ist.

7. Zusammensetzung nach Anspruch 2, wobei das Peptidhormon ein Wachstumshormon ist.

8. Zusammensetzung nach Anspruch 2, wobei die hämatopoetische Faktor das Erythropoetin ist.

9. Zusammensetzung nach Anspruch 2, wobei der auf das Immunsystem einwirke Faktor ein Impfstoff ist.

10. Zusammensetzung nach Anspruch 1, wobei die Menge des pharmazeutischen Wassers im Bereich von 1,0 bis 99,9%, bezogen auf das Gesamtgewicht der Zusammensetzung, liegt.

11. Zusammensetzung nach Anspruch 10, wobei die Menge des pharmazeutischen Wassers im Bereich von 50,0 bis 99,9%, bezogen auf das Gesamtgewicht der Zusammensetzung, liegt.

## Revendications

1. Composition thérapeutique comprenant une quantité efficace d'un agent biologiquement actif et d'une eau pharmaceutique, la teneur en eau de la composition étant de l'ordre de 99,760 à 99,999% de l'isotopologue léger ¹H₂¹⁶O et jusqu'à 100 % des isotopologues résiduels ¹H₂¹⁷O, ¹H₂¹⁸O, ¹H²H¹⁶O, ¹H²H¹⁷O, ¹H²H¹⁸O, ²H₂¹⁶O, ²H₂¹⁷O, et ²H₂¹⁸O,
dans laquelle l'agent biologiquement actif est choisi dans le groupe constitué par les peptides physiologiquement actifs, les protéines physiologiquement actives, et les médicaments choisis dans le groupe constitué par les agents analgésiques, les agents anesthésiques, les agents antiacné, les agents anti-âge, les agents antiallergiques, les agents antianémiques, les agents antiangineux, les agents anxiolytiques, les agents antiarythmiques, les agents antiasthmatiques, les agents antibactériens, les agents anticancéreux, les agents anticholinergiques, les agents anticoagulants, les agents anticonvulsivants, les agents antidépresseurs, les agents antidiabétiques, les agents antidiarrhéiques, les agents antidiurétiques, les antidotes, les agents antidyskinétiques, les agents antidysmérorrhéiques, les agents antiémétiques, les agents antifibrinolytiques, les agents antifibrinolytiques, les agents antifongiques, les agents antiglaucome, les agents antihémorragiques, les agents antihistaminiques, les agents antihypercalcémiques, les agents antihyperglycémiques, les agents antihyperlipidémiques, les agents antihypertenseurs, les agents antihyperthyroïdiens, les agents antihyperuricémiques, les agents antihypocalcémiques, les agents antihypoglycémiques, les agents antihypotenseurs, les agents anti-inflammatoires, les agents contre la maladie de Kawasaki, les agents antipaludéens, les agents antiméthémoglobinémiques, les agents antimicrobiens, les agents antimigraineux, les agents antimyasténiques, les agents antinéoplasiques, les agents antinévralgiques, les agents antineutropéniques, les agents antipanique, les agents contre la polyneuropathie, les agents antiprotozoaires, les agents antipsoriatiques, les agents antipsychotiques, les agents antipyrétiques, les agents antirhumatismaux, les agents antiséborrhéiques, les agents antispasmodiques, les agents antithrombotiques, les agents anti-tremblement, les agents ulcéreux, les agents antivertigineux, les agents antiviraux, les agents antirides, les stimulants de l'appétit, les anorexiants, les agents prophylactiques contre l'asthme, les inhibiteurs de la résorption osseuse, les bronchodilatateurs, les agents contraceptifs, les corticostéroïdes, les agents de dépigmentation, les agents désinfectants, les agents diurétiques, les composants d'un solution de dialyse, les oestrogènes, les agents favorisant la croissance capillaire, les agents inhibant la croissance capillaire, les stimulants hémapoïétiques, les agents de traitement de l'hépatite, les antagonistes des récepteurs H2 de l'histamine, les hormones, les agents de traitement de l'insulinorésistance, les agents de traitement de la résistance aux interférons, les immunosuppresseurs, les agents de traitement de l'impotence, les agents kératolytiques, les agents myotiques, les agents mucolytiques, les agents mydriatiques, les agents de prévention ou de traitement d'un infarctus du myocarde, les agents du blocage neuromusculaire, les agents de prévention ou de traitement de l'ostéoporose, les agents sédatifs, les relaxants musculaires, les agents de blanchiment de la peau, les agents sympathomimétiques, les agents thrombolitiques, les vaccins, les vasodilatateurs, les améliorateurs de cicatrisation, les révulsifs, les extraits végétaux, les rétinoïdes, et les bioflavonoïdes.

2. Composition selon la revendication 1, dans laquelle le peptide physiologiquement actif ou la protéine physiologiquement active est choisi(e) dans le groupe constitué par les cytokines, les hormones peptidiques, les facteurs de croissance, les facteurs agissant sur le système cardiovasculaire, les facteurs agissant sur les systèmes nerveux central et périphérique, les facteurs agissant sur les électrolytes de l'humeur, les facteurs hématopoïétiques, les facteurs agissant sur les os et le squelette, les facteurs agissant sur le système gastrointestinal, les facteurs agissant sur le système immunitaire, les facteurs agissant sur les organes génitaux, et les enzymes.

3. Composition selon la revendication 2, dans laquelle la cytokine est l'interféron alpha.

4. Composition selon la revendication 2, dans laquelle l'hormone peptidique est l'insuline.

5. Composition selon la revendication 2, dans laquelle le facteur de croissance est un facteur de croissance de type insuline.

6. Composition selon la revendication 2, dans laquelle l'hormone peptidique est la calcitonine.

7. Composition selon la revendication 2, dans laquelle l'hormone peptidique est une hormone de croissance.

8. Composition selon la revendication 2, dans laquelle le facteur hématopoïétique est l'érythropoïétine.

9. Composition selon la revendication 2, dans laquelle le facteur agissant sur le système immunitaire est un vaccin.

10. Composition selon la revendication 1, dans laquelle la quantité d'eau pharmaceutique est de l'ordre de 1,0 à 99,9 %, basée sur le poids total de la composition.

11. Composition selon la revendication 10, dans laquelle la quantité d'eau pharmaceutique est de l'ordre de 50,0 à 99,9 %, basée sur le poids total de la composition.
